# EUROPEAN PATENT SPECIFICATION

(11) **EP 3 173 399 B1**
(45) Date of publication and mention of the grant of the patent: **15.08.2018**
(21) Application number: 16199414.0
(22) Date of filing: 17.11.2016
(51) Int. Cl.: C07C 209/48, C07C 211/14

(54) **METHOD FOR PREPARING N,N'-BIS(3-AMINOPROPYL)-1,2-ETHYLENEDIAMINE**
VERFAHREN ZUR HERSTELLUNG VON N,N'-BIS(3-AMINOPROPYL)-1,2-ETHYLENEDIAMIN
PROCÉDÉ DE PRÉPARATION DE N,N '-BIS(3-AMINOPROPYL)-1,2-ÉTHYLÉNÉDIAMINE

(30) Priority: 26.11.2015 TW 104139370
(43) Date of publication of application: 31.05.2017
(73) Proprietor: Nan Ya Plastics Corporation, Taipei 105 (TW)
(72) Inventor: LIAO, Te-Chao, 105 Taipei (TW); CHUANG, Jung-Jen, 105 Taipei (TW); HUANG, Zhi-Ming, 105 Taipei (TW); CHEN, Shun-Chi, 105 Taipei (TW); LIN, Tzu-Chiang, 105 Taipei (TW)
(74) Representative: Beck & Rössig European Patent Attorneys

(56) References cited:
- US-A1- 2008 194 857
- MERVYN ISRAEL ET AL: "Analogs of Spermine and Spermidine. I. Synthesis of Polymethylenepolyamines by Reduction of Cyanoethylated [alpha],[iota]-Alkylenediamines 1,2", JOURNAL OF MEDICINAL CHEMISTRY, vol. 7, no. 6, 1 November 1964 (1964-11-01), pages 710-716, XP055354696, US ISSN: 0022-2623, DOI: 10.1021/jm00336a006

## Description

### BACKGROUND OF THE INVENTION

### 1. Field of the Invention

The present invention relates to N,N'-bis(3-aminopropyl)-1,2-ethylenediamine, and more particularly to a method for preparing N,N'-bis(3-aminopropyl)-1,2-ethylenediamine with improved yield.

### 2. Description of Related Art

Conventionally, synthesis of N,N'-bis(3-aminopropyl)-1,2-ethylenediamine (hereinafter shortened as BAEDA) is achieved using a monol-based solvent and a metal catalyst containing cobalt or nickel. However, usage of monol-based solvent leads to many by-products that have negative impact on the quality of the final products. As a result, the yield of BAEDA is limited and costly purification systems may be required to refine the final products.

For example, US 5,434,262 discloses a process where BAEDA and ethanol as a solvent perform hydrogenation in the presence of Raney nickel catalyst to get the expected product with a yield of 60%.

As another example, GB 2067191B discloses a method of hydrogenating polynitriles to polyamines using a pelleted cobalt-zinc hydrogenation catalyst.

A further instance is US 2008/0194857A1, which discloses a method of using Raney Cobalt as a catalyst to prepare BAEDA in the presence of isopropanol whose product gave a 98.16% yield as determined by gas chromatography (GC).

Additionally, it is known in the art to use a monol-based solvent (ethanol, isopropanol or methanol) and Raney nickel as a catalyst for hydrogenation. As a result, ethanol and isopropanol are preferred solvents for preparing BAEDA through hydrogenation, which contribute to a yield of 95.80%.

### SUMMARY OF THE PRESENT INVENTION

To break the stereotype that BAEDA is conventionally made in the presence of a monol-based solvent as a solvent in hydrogenation, the present invention discloses a method for preparing BAEDA with high selectivity, which uses dipropylene glycol dimethyl ether (PM) as a solvent and uses a cobalt-manganese-aluminum catalyst (hereinafter referred to as Co-Mn-Al catalyst) to improve the yield of BAEDA to 98.85-99.49% while effectively suppressing generation of by-products.

The disclosed method for preparing BAEDA comprises the following synthesis steps:
a) taking N,N'-bis(2-cyanoethyl)-1,2-ethylenediamine (hereinafter shortened as BCNEDA) as the reactant (the base material);
b) in a high-pressure reactor, adding 1.00-2.00wt% of a Co-Mn-Al catalyst and 33.21-38.53wt% of a PM-LiOH solution (0.01mol) based on the weight of the base material, wherein the PM-LiOH solution is prepared by dissolving 1 mol% of LiOH based on the BCNEDA reactant into 29-35wt% of a glycol-ether-based solution based on the total reactant weight, in which the glycol-ether-based solution is prepared by mixing 89-92wt% of a dipropylene glycol dimethyl ether (PM) solvent and 8-11wt% of water;
c) under high speed stirring injecting BCNEDA gradually into the reactor using a syringe pump within 3 hours in an environment of 110-130°C and 700-900psi (4.8-6.2MPa);
d) allowing reaction to continuously take place at 120°C; using gas chromatography (GC) to verify that conversion of BCNEDA reaches 100% and finishing the reaction if so. Analysis of the product of the reaction confirms that the yield of BAEDA is 98.85-99.49%.

### DETAILED DESCRIPTION OF THE INVENTION

The method for preparing BAEDA according to present invention takes a certain amount of BCNEDA as the reactant to perform hydrogenation synthesis in the presence of a PM solution of a specific formula and the final reactant obtained after the reaction improves the yield of BAEDA to 98.85-99.49%.

The disclosed method for preparing BAEDA comprises the following synthesis steps:
a) adding a Co-Mn-Al catalyst with a specific formula into a PM-LiOH solution;
b) transferring the solution of Step a) into a 300mL agitator-equipped high-pressure reactor that is set with a temperature of 120°C and a hydrogen pressure of 800psi (5.5MPa), and after the system becomes steady, pumping 93.5g of BCNEDA into the reactor for hydrogenation; and
c) allowing the reaction to take place continuously at the temperature of 120°C and the hydrogen pressure of 800psi (5.5MPa), using GC to verify that conversion of BCNEDA reaches 100% and finishing the reaction if so, thereby obtaining a mixture containing BAEDA.

The key technology of the disclosed method for preparing BAEDA relies on using the specially formulated Co-Mn-Al catalyst as the catalyst and the PM-LiOH solution for synthesis, so as to improve the yield of BAEDA to 98.85-99.49% and effectively suppress generation of by-products.

The specially formulated Co-Mn-Al catalyst is uses in an amount of 1.00-2.00wt% based on the weight of the BCNEDA reactant.

The PM-LiOH solution is prepared by dissolving 1 mol% of LiOH based on the BCNEDA reactant into 29-35wt% of the glycol ether solution based on the total reactant weight. The glycol-ether-based solution is preferably a mixed solution of a dipropylene glycol dimethyl ether (PM) solvent and water, wherein water takes 8-11wt% in the mixed solution. Water is added for helping lithium hydroxide to dissolve and distribute across the PM. Lithium hydroxide is used as a catalyst promoter for activating the catalyst and takes 1 mol% of BCNEDA.

### Example 1:

In a 300mL agitator-equipped high-pressure reactor, 1.000g of a Co-Mn-Al catalyst (Kawaken OFT-55), 0.065g of LiOH, 3.230g of water and 27.752g of dipropylene glycol dimethyl ether (PM) solution were introduced. In an environment of a constant temperature of 120°C and a hydrogen pressure of 800psi (5.5MPa), 93.500 g BCNEDA was pumped into the reactor.

After the BCNEDA was pumped in, the mixture was allowed to continue reaction at 120°C. Gas chromatography (hereinafter shortened as GC) was used to confirm that conversion of BCNEDA reached 100%.

After the reaction, a final reaction product was analyzed in terms of composition, and the results are shown in Table 1. The yield of BAEDA is 99.49%.

### Example 2:

The process is similar to that of Example 1, but had the amount of the Co-Mn-Al catalyst (Kawaken OFT-55) increased to 1.500g.

After the reaction, a final reaction product was analyzed in terms of composition, and the results are shown in Table 1. The yield of BAEDA is 99.00%.

### Example 3:

The process is similar to that of Example 1, but a different Co-Mn-Al catalyst (Kawaken OFT-MS) was used.

After the reaction, a final reaction product was analyzed in terms of composition, and the results are shown in Table 1. The yield of BAEDA is 99.15%.

### Example 4:

The process is similar to that of Example 3, but a different Co-Mn-Al catalyst (Kawaken OFT-MS) was used.

After the reaction, a final reaction product was analyzed in terms of composition, and the results are shown in Table 1. The yield of BAEDA is 98.85%.

### Comparative Example 1:

In a 180mL high-pressure reactor, 1.0700g of Raney cobalt 2724 catalyst, 0.2520g of LiOH•H2O, 1.0000g of H2O, 29.8g of isopropanol were introduced. In an environment of a constant temperature of 120°C and a hydrogen pressure of 800psi (5.5MPa), 100.4 g BCNEDA was pumped into the reactor.

After the BCNEDA was pumped in, the mixture was allowed to continue reaction at 120°C. Gas chromatography (hereinafter shortened as GC) was used to confirm that conversion of BCNEDA reached 100%.

After the reaction, a final reaction product was analyzed in terms of composition, and the results are shown in Table 1. The yield of BAEDA is 98.16%.

### Comparative Example 2:

In a 300mL high-pressure reactor, 12.000g of Raney nickel catalyst, 100.00g of ammonia anhydrous, 20.000g of the PM solvent were introduced. In an environment of a constant temperature of 120°C and a hydrogen pressure of 800psi (5.5MPa), 93.500 g of BCNEDA was pumped into the reactor.

After the BCNEDA was pumped in, the mixture was allowed to continue reaction at 120°C. Gas chromatography (hereinafter shortened as GC) was used to confirm that conversion of BCNEDA reached 100%.

After the reaction, a final reaction product was analyzed in terms of composition, and the results are shown in Table 1. The yield of BAEDA is 85.10%.

### Comparative Example 3:

In a 1000mL high-pressure reactor, 25.000g of Raney nickel catalyst, 50.00g of ammonia anhydrous and 20.000g of methanol were introduced. In an environment of a constant temperature of 65°C and a hydrogen pressure of 3.5-4.5MPa, 400 g of BCNEDA that contained 118.383g of methanol was pumped into the reactor.

After the BCNEDA was pumped in, the mixture was allowed to continue reaction at 55-65°C. Gas chromatography (hereinafter shortened as GC) was used to confirm that conversion of BCNEDA reached 100%.

After the reaction, a final reaction product was analyzed in terms of composition, and the results are shown in Table 1. The yield of BAEDA is 85.80%.

### Comparative Example 4:

In a 1000mL high-pressure reactor, 25.000g of Raney nickel catalyst, 2.000g of sodium hydroxide and 20.000g of ethanol were introduced. In an environment of a constant temperature of 55°C and a hydrogen pressure of 1.5MPa, 400 g of BCNEDA that contained 118.383g of ethanol was pumped into the reactor.

After the BCNEDA was pumped in, the mixture was allowed to continue reaction at a temperature of 60-70°C and a hydrogen pressure of 1.5-2.0MPa. Gas chromatography (hereinafter shortened as GC) was used to confirm that conversion of BCNEDA reached 100%.

After the reaction, a final reaction product was analyzed in terms of composition, and the results are shown in Table 1. The yield of BAEDA is 95.80%.

### Comparative Example 5:

The process is similar to that of Example 1, but the amount of the Co-Mn-Al catalyst (Kawaken OFT-55) was reduced to 0.500g.

After the reaction, the product was analyzed in terms of composition, and the results are shown in Table 1. The yield of BAEDA is 79.31%.

### Comparative Example 6:

The process is similar to that of Comparative Example 5, but a different Co-Mn-Al catalyst (Kawaken OFT-MS) was used.

After the reaction, the product was analyzed in terms of composition, and the results are shown in Table 1. The yield of BAEDA is 77.43%.

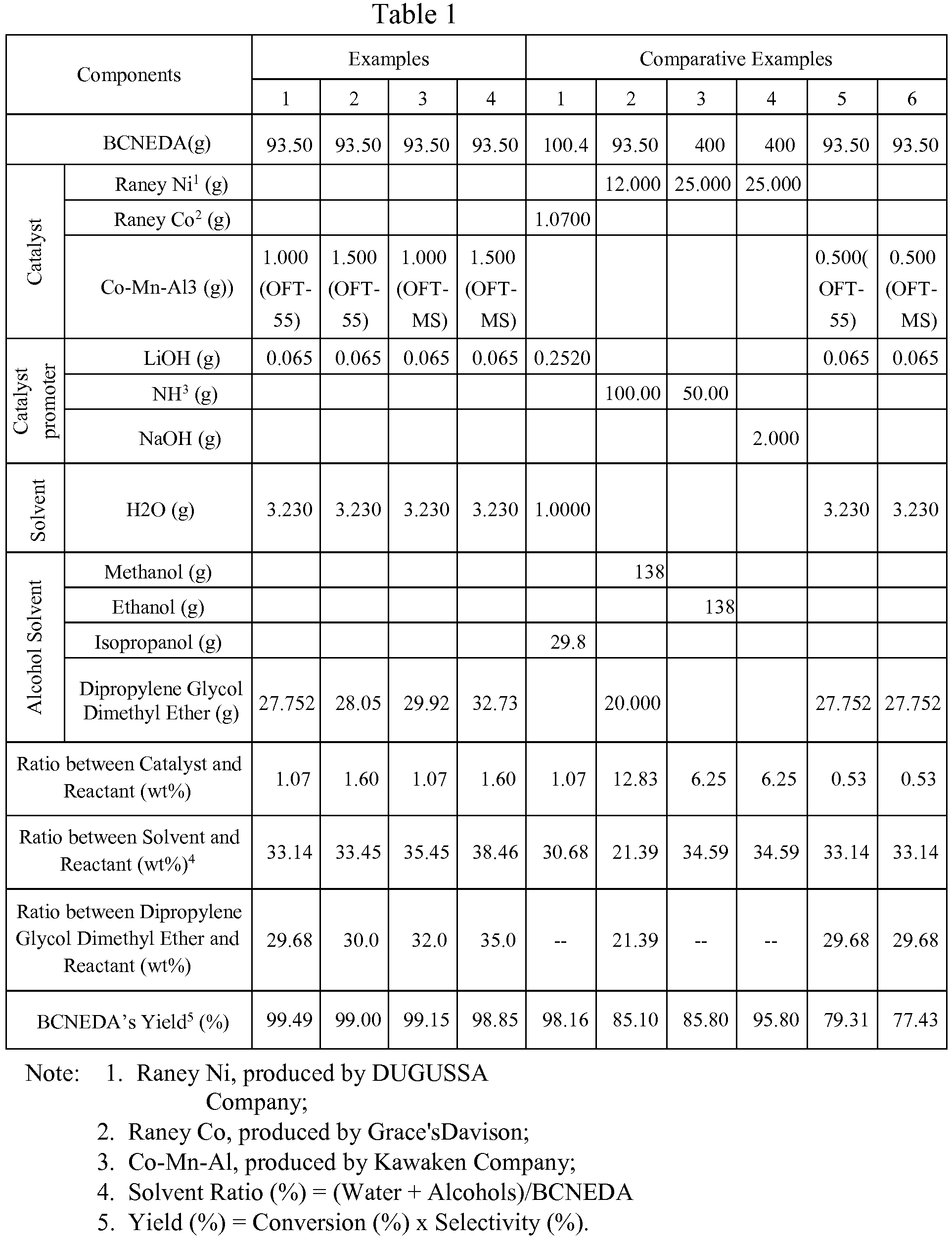

### Conclusion:

1. As learned from Examples 1-4, in the process of preparing BAEDA, by using a glycol-ether-based solvent such as dipropylene glycol dimethyl ether (PM) of a certain ratio to perform hydrogenation in the presence of a specially formulated Co-Mn-Al catalyst, the reaction was made safe and the yield of BAEDA reached 98.85-99.49%.
2. As demonstrated in Comparative Examples 1-4, in the process of preparing BAEDA, use of a monol-based solvent with Raney Ni or Raney Co as the catalyst for hydrogenation, the yield of BAEDA could only achieve 85.10-98.16%.
3. As learned from Comparative Examples 5-6, in the process of preparing BAEDA, by using a glycol-ether-based solvent such as PM of a certain ratio to perform hydrogenation in the presence of 0.53wt% of Co-Mn-Al catalyst, the yield of BAEDA was 77.43% and 79.31% respectively. The amount of the catalyst was too low to improve the yield of BAEDA.

## Claims

1. A method for preparing N,N'-bis(3-aminopropyl)-1,2-ethylenediamine, comprising:
a) in a high-pressure reactor, adding 1.00-2.00wt% of a Co-Mn-Al catalyst and 33.21-38.53wt% of a dipropylene glycol dimethyl ether-LiOH (0.01mol) solution based on a weight of N,N'-bis(2-cyanoethyl)-1,2-ethylenediamine (BCNEDA) as a base material;
b) in an environment of a temperature of 110-130°C and a pressure of 700-900psi (4.8-6.2MPa), gradually pumping BCNEDA into the reactor under high speed stirring using a syringe pump within 3 hours;
c) after pumping, allowing the mixture to continue reaction at 120°C;
d) using gas chromatography (GC) to verify that conversion of BCNEDA reaches 100% and finishing the reaction; and
e) a yield of N,N'-bis(3-aminopropyl)-1,2-ethylenediamine (BAEDA) ranging between 98.85% and 99.49% is obtained.

2. The method of claim 1, wherein the Co-Mn-Al catalyst is added in an amount of 1.0wt%.

3. The method of claim 1 or 2, wherein N,N'-bis(2-cyanoethyl)-1,2-ethylenediamine is continuously introduced for hydrogenation.

4. The method of any of claims 1 to 3, wherein the pressure for synthesis is 800psi (5.5MPa).

5. The method of any of claims 1 to 4, wherein BCNEDA is gradually pumped into the reactor at the temperature of 120°C.

6. The method of any of claims 1 to 5, wherein the N,N'-bis(2-cyanoethyl)-1,2-ethylenediamine is pumped in over a duration less than three hours.

7. The method of any of claims 1 to 6, wherein the dipropylene glycol dimethyl ether is added in an amount of 33wt% based on the weight of the BCNEDA reactant.

8. The method of any of claims 1 to 7, wherein the dipropylene glycol dimethyl ether -LiOH solution is prepared by dissolving 1 mol% of LiOH based on the BCNEDA reactant into 29-35wt% of a glycol-ether-based solution based on the weight of the BCNEDA reactant, and the glycol-ether-based solution is prepared by mixing 89-92wt% of a dipropylene glycol dimethyl ether (PM) solvent with 8-11wt% of water.

## Patentansprüche

1. Verfahren zur Herstellung von N,N'-bis(3-Aminopropyl)-1,2-Ethylendiamin, umfassend:
a) in einem Hochdruckreaktor, Hinzufügen von 1,00 - 2,00 Gew.-% eines Co-Mn-Al-Katalysators und von 33,21 - 38,53 Gew.-% einer Dipropylenglykoldimethylether-LiOH-Lösung (0,01 mol) basierend auf einem Gewicht von N,N'-bis(2-Cyanoethyl)-1,2-Ethylendiamin (BCNEDA) als Basismaterial;
b) in einer Umgebung mit einer Temperatur von 110 - 130 °C und einem Druck von 700 -900 psi (4,8 - 6,2 MPa), allmähliches Pumpen von BCNEDA in den Reaktor unter Hochgeschwindigkeitsrühren unter Verwendung einer Spritzenpumpe innerhalb von 3 Stunden;
c) nach dem Pumpen, Zulassen der Fortsetzung der Reaktion der Mischung bei 120 °C;
d) Verwenden von Gaschromatographie (GC), um zu verifizieren, dass die Umwandlung von BCNEDA 100 % erreicht hat und Beenden der Reaktion; und
e) ein Ertrag von N,N'-bis(3-Aminopropyl)-l,2-Ethylendiamin (BAEDA) im Bereich zwischen 98,85 % und 99,49 % erreicht wird.

2. Verfahren nach Anspruch 1, wobei der Co-Mn-Al-Katalysator in einer Menge von 1,0 Gew.-% hinzugefügt wird.

3. Verfahren nach Anspruch 1 oder 2, wobei N,N'-bis(2-Cyanoethyl)-1,2-Ethylendiamin kontinuierlich für die Hydrierung eingeleitet wird.

4. Verfahren nach einem der Ansprüche 1 bis 3, wobei der Druck für die Synthese 800 psi (5,5 MPa) beträgt.

5. Verfahren nach einem der Ansprüche 1 bis 4, wobei BCNEDA allmählich in den Reaktor bei einer Temperatur von 120 °C gepumpt wird.

6. Verfahren nach einem der Ansprüche 1 bis 5, wobei das N,N'-bis(2-Cyanoethyl)-1,2-Ethylendiamin über eine Dauer von weniger als drei Stunden eingepumpt wird.

7. Verfahren nach einem der Ansprüche 1 bis 6, wobei der Dipropylenglykoldimethylether in einer Menge von 33 Gew.-% basierend auf dem Gewicht des BCNEDA-Reaktanten hinzugefügt wird.

8. Verfahren nach einem der Ansprüche 1 bis 7, wobei die Dipropylenglykoldimethylether-LiOH-Lösung durch Auflösen von 1 mol% LiOH basierend auf dem BCNEDA-Reaktanten in 29 - 35 Gew.-% einer glykoletherbasierten Lösung basierend auf dem Gewicht des BCNEDA-Reaktanten hergestellt wird, und die glykoletherbasierte Lösung durch Mischen von 89 - 92 Gew.-% eines Dipropylenglykoldimethylether- (PM) Lösungsmittels mit 8 - 11 Gew.-% Wasser hergestellt wird.

## Revendications

1. Procédé de préparation de N,N'-bis(3-aminopropyl)1,2-éthylènediamine comprenant :
a) dans un réacteur haute pression, ajout de 1 à 2% en poids d'un catalyseur Co-Mn-Al et 33,21 à 33,53% en poids d'une solution de LiOH (0,01 mol) à l'éther diméthylique de dipropylèneglycol sur la base de N,N'-bis(2-cyanoéthyl)-1,2-éthylènediamine (BCNEDA) comme matière de base ;
b) dans un environnement d'une température de 110 à 130°C et une pression de 700 à 900 psi (4,8 à 6,2 MPa), pompage graduel de BCNDA dans le réacteur en remuant à haute vitesse en utilisant une pompe à seringue dans les 3 heures ;
c) après pompage, faire continuer la réaction pour le mélange à 120°C ;
d) utilisation de la chromatographie gazeuse (GC) pour vérifier que la conversion de BCNEDA atteint 100% et finition de la réaction et
e) un rendement de N,N'-bis(3-aminopropyl)1,2-éthylènediamine (BAEDA) de l'ordre de 98,85% et 99,49% est obtenu.

2. Procédé selon la revendication 1, le catalyseur Co-Mn-Al étant ajouté en une quantité de 1,0 % en poids.

3. Procédé selon la revendication 1 ou 2, le N,N'-bis(3-aminopropyl)1,2-éthylènediamine étant introduit en continu pour hydrogénation.

4. Procédé selon l'une quelconque des revendications 1 à 3, la pression pour la synthèse étant de 800 psi (5,5 MPa).

5. Procédé selon l'une quelconque des revendications 1 à 4, le BCNEDA étant pompé graduellement dans le réacteur à la température de 120°C.

6. Procédé selon l'une quelconque des revendications 1 à 5, le N,N'-bis(3-aminopropyl)1,2-éthylènediamine étant pompé pendant une durée de moins de 3 heures.

7. Procédé selon l'une quelconque des revendications 1 à 6, l'éther diméthylique de dipropylèneglycol étant ajouté en une quantité de 33% en poids basé sur le poids du réactif de BCNEDA.

8. Procédé selon l'une quelconque des revendications 1 à 7, la solution de LiOH et d'éther diméthylique de dipropylèneglycol étant préparée en dissolvant 1% molaire de LiOH basé sur le réactif de BCNEDA dans 29 à 35% en poids d'uen solution à base d'éther de glycol sur la base du poids du réactif de BCNEDA et la solution à base d'éther de glycol étant préparée en mélangeant 89 à 92% en poids d'un solvant à l'éther diméthylique de dipropylèneglycol (PM) avec 8 à 11% en poids d'eau.
